# EUROPÄISCHE PATENTSCHRIFT

(11) **EP 3 050 856 B1**
(45) Veröffentlichungstag und Bekanntmachung des Hinweises auf die Patenterteilung: **29.05.2019**
(21) Anmeldenummer: 15153296.7
(22) Anmeldetag: 30.01.2015
(51) Int. Cl.: C03C 3/078, C03C 3/097, C03C 3/112, C03C 4/00, C03C 8/02, C03C 8/06, C03C 8/08, C03C 10/00, A61K 6/027

(54) **Lithiumsilikat-Diopsid-Glaskeramik**
Lithium silicate diopside glass ceramic
Vitrocéramique en diopside-lithium-silice

(43) Veröffentlichungstag der Anmeldung: 03.08.2016
(73) Patentinhaber: Ivoclar Vivadent AG, 9494 Schaan (LI)
(72) Erfinder: Rampf, Markus, 8853 Lachen (CH); Ritzberger, Christian, 9472 Grabs (CH); Dittmer, Marc, 6800 Feldkirch (AT); Höland, Wolfram, 9494 Schaan (LI); Schweiger, Marcel, 7000 Chur (CH)
(74) Vertreter: Uexküll & Stolberg

(56) Entgegenhaltungen:
- EP-A1- 1 167 311
- WO-A1-2015/173394
- WO-A2-2013/053864
- SALMAN S M ET AL: "Crystallization characteristics and physico-chemical properties of the glasses based on Li2O-CaO-SiO2 eutectic (954<o>C) system containing magnesium oxide", CERAMICS INTERNATIONAL, ELSEVIER, AMSTERDAM, NL, Bd. 34, Nr. 8, 1. Dezember 2008 (2008-12-01), Seiten 1819-1828, XP025479744, ISSN: 0272-8842, DOI: 10.1016/J.CERAMINT.2007.06.008 [gefunden am 2008-10-01]
- SALMAN S M ET AL: "Crystallization behaviour and properties of multicomponent strontium-Containing lithia calcia silicate glasses", CERAMICS INTERNATIONAL, ELSEVIER, AMSTERDAM, NL, Bd. 36, Nr. 8, 1. Dezember 2010 (2010-12-01), Seiten 2307-2314, XP027392072, ISSN: 0272-8842 [gefunden am 2010-08-03]
- SALMAN S M ET AL: "The influence of Al2O3, MgO and ZnO on the crystallization characteristics and properties of lithium calcium silicate glasses and glass-ceramics", MATERIALS CHEMISTRY AND PHYSICS, ELSEVIER SA, SWITZERLAND, TAIWAN, REPUBLIC OF CHINA, Bd. 112, Nr. 3, 20. Dezember 2008 (2008-12-20), Seiten 945-953, XP025656805, ISSN: 0254-0584, DOI: 10.1016/J.MATCHEMPHYS.2008.07.022 [gefunden am 2008-08-08]

## Beschreibung

Die Erfindung betrifft Lithiumsilikat-Diopsid-Glaskeramik, die sich insbesondere zum Einsatz in der Zahnheilkunde, bevorzugt zur Herstellung von dentalen Restaurationen, eignet sowie Vorstufen zur Herstellung der Glaskeramik.

Glaskeramiken mit einer Lithiumsilikat-Kristallphase und deren Verwendung in Dentalprodukten sind aus dem Stand der Technik bekannt. Beispielsweise beschreibt die EP 1 505 041 Lithiumsilikat-Glaskeramiken, die in Form von Lithiummetasilikat-Glaskeramiken mittels CAD/CAM-Verfahren zu den gewünschten Dentalrestaurationen verarbeitet werden, wobei eine anschließende Wärmebehandlung zur Umwandlung der Lithiummetasilikat (Li₂SiO₃)-Phase in Lithiumdisilikat (Li₂Si₂O₅) -Phase und damit zur Ausbildung hochfester Lithiumdisilikat-Glaskeramik führt. Eine maschinelle Bearbeitung der Glaskeramik nach Ausbildung der Lithiumdisilikat-Phase ist insbesondere aufgrund deren hoher Festigkeit zeitaufwendig und mit hohem Werkzeugverschleiß verbunden.

Glaskeramiken, die Diopsid, CaMgSi₂O₆, als Kristallphase enthalten, sind bekannt. Diopsid kann als Nebenphase in Amphibol-Glaskeramiken (Höland, Beall, "Glass-Ceramic Technology", Wiley, USA, 2. Auflage, 2012, S. 151), in Apatit-Glaskeramiken (ebenda, S. 164) oder in Basalt-Glaskeramiken (ebenda, S. 186) entstehen.

S. M. Salman et al., Ceramics International 2008, 34(8), 1819-1828 und Ceramics International 2010, 36(8), 2307-2314 beschreiben die Bildung von Glaskeramiken, die Lithiumdisilikat oder Lithiummetasilikat als Hauptkristallphase und unter anderem Diopsid als weitere Kristallphase enthalten können.

Aus der WO 2009/140632 (Ohio State University) sind Lanthanoxiddotierte bioaktive Glaskeramiken als Komponente von zum Beispiel Dentalrestaurationen bekannt, die als eine Kristallphase Diopsid enthalten können.

Aus der US 4,560,666 (Hoya Corporation) sind bioaktive Glaskeramiken bekannt, die Apatit und Diopsid enthalten können und als Material für künstlichen Knochen oder künstliche Zahnwurzeln Verwendung finden sollen. Die WO 2012/172316 (University of Sheffield) offenbart keramisches Material für Dentalrestaurationen, das Diopsid und Leucit als Kristallphasen enthält. Die EP 1 132 056 (Tokuyama Corporation) beschreibt ein Verfahren zur Herstellung von keramischen Zahnkronen unter Verwendung einer Diopsid-haltigen Glaskeramik.

Die US 4,643,982 (Hoya Corporation) beschreibt hochfeste Anorthit-Glaskeramiken, die Apatit- oder Calciumphosphat-Kristalle sowie gegebenenfalls weitere Kristallphasen wie Diopsid enthalten können. Aufgrund des Vorliegens mehrerer Kristallphasen zeichnen sich die Glaskeramiken durch eine hohe Opazität aus. Aus diesem Grund sind sie für ästhetisch anspruchsvolle Dentalrestaurationen nicht geeignet. Sie sind vielmehr als Implantatmaterial oder Werkstoff für Wurzelstifte vorgesehen, für die keine besonderen optischen Eigenschaften notwendig sind.

Die US 5,066,619 (Hoya Corporation) beschreibt Glaskeramiken mit Glimmerphase und mindestens einer weiteren Kristallphase ausgewählt aus Enstatit, Akermanit, Diopsid, Anorthit und Richterit, die zur Herstellung von Zahnkronen geeignet sein sollen. Aus der US 5,246,889 (Hoya Corporation) sind ebenfalls Glimmer-Glaskeramiken bekannt, die Zirkonoxid als weitere Kristallphase aufweisen. Bei manchen der beschriebenen Glaskeramiken können auch Kristallphasen aus Enstatit, Akermanit, Diopsid, Anorthit, Richterit und Forsterit auftreten.

Die US 4,871,384 und die US 5,232,878 (beide Hoya Corporation) beschreiben bioaktive Apatit-Glaskeramiken, die als weitere Kristallphase u.a. Diopsid enthalten können. Die Glaskeramiken sind hauptsächlich als Knochenersatzmaterial vorgesehen.

Die US 5,356,436 und die US 5,711,763 (beide TDK Corporation) offenbaren Keramikmaterialien zum Ersatz von hartem Körpergewebe, wobei die Materialien Wollastonit, Diopsid oder eine Kombination dieser Kristallphasen aufweisen.

Die US 2005/0079226 (Pentax Corporation) beschreibt bioaktives Glas, das als Sinterhilfsmittel für Knochenersatzmaterialien eingesetzt werden kann und nach Kristallisation Wollastonit- und Diopsid-Kristallphasen enthalten kann.

Die bekannten Materialien weisen allerdings eine Reihe von Nachteilen auf. Bei ihnen kann in vielen Fällen die Transluzenz nicht über einen breiten Bereich eingestellt werden, wie es für vielseitig einsetzbare Dentalmaterialien wünschenswert ist. Darüber hinaus ist bei ihnen häufig eine einfache maschinelle Bearbeitung nicht möglich. Zudem erweist sich ihre Festigkeit häufig als nicht ausreichend, um ihren Einsatz als restauratives Dentalmaterial zu gestatten.

Der Erfindung liegt die Aufgabe zugrunde, eine Glaskeramik zur Verfügung zu stellen, die über gute optische Eigenschaften, insbesondere eine steuerbare Transluzenz, sowie gute mechanische Eigenschaften verfügt und damit als restauratives Dentalmaterial verwendet werden kann. Die Glaskeramik soll darüber hinaus in einfacher und schneller Weise durch maschinelle Bearbeitung, z.B. mittels CAD/CAM-Verfahren, zu dentalen Restaurationen verarbeitbar sein. Diese einfache Bearbeitung soll insbesondere auch nach möglichst vollständiger Kristallisation der gewünschten Kristallphasen möglich sein.

Diese Aufgabe wird durch die Verwendung der Lithiumsilikat-Diopsid-Glaskeramik nach den Ansprüchen 1 bis 19 gelöst. Gegenstand der Erfindung sind ebenfalls die Verwendung des Ausgangsglases nach Anspruch 20, die Lithiumsilikat-Diopsid-Glaskeramik nach Ansprüchen 21 und 22, das Ausgangsglas nach Ansprüchen 23 und 24 sowie das Verfahren nach Anspruch 25.

Die erfindungsgemäß verwendete Lithiumsilikat-Diopsid-Glaskeramik zeichnet sich dadurch aus, dass sie Lithiumsilikat als Hauptkristallphase und Diopsid als weitere Kristallphase enthält.

Diese Glaskeramik zeigt überraschenderweise eine vorteilhafte Kombination von für ein restauratives Dentalmaterial wünschenswerten mechanischen und optischen Eigenschaften und sie kann zudem in einer für ein Dentalmaterial vorteilhaften Weise in die gewünschte Form, zum Beispiel einer Dentalrestauration, wie einer Krone, gebracht werden.

Die erfindungsgemäß verwendete Glaskeramik enthält insbesondere 53,0 bis 75,0, vorzugsweise 54,0 bis 74,0 und besonders bevorzugt 58,0 bis 70,0 Gew.-% SiO₂.

Es ist weiter bevorzugt, dass die Glaskeramik 10,0 bis 23,0, insbesondere 11,0 bis 20,0 und besonders bevorzugt 11,0 bis 16,0 Gew.-% Li₂O enthält.

Das molare Verhältnis von SiO₂ zu Li₂O beträgt insbesondere 2,0 bis 3,0.

Des Weiteren ist es bevorzugt, dass die Glaskeramik 1,0 bis 13,0, insbesondere 1,0 bis 9,0 und besonders bevorzugt 1,0 bis 6,0 Gew.-% CaO enthält.

Die Glaskeramik enthält vorzugsweise 1,0 bis 12,0, insbesondere 2,0 bis 9,0 und besonders bevorzugt 2,0 bis 5,0 Gew.-% MgO enthält.

Das molare Verhältnis von CaO zu MgO beträgt vorzugsweise 0,5 bis 2,0, besonders bevorzugt 0,8 bis 1,2 und ganz besonders bevorzugt etwa 1,0.

Weiter ist eine Glaskeramik bevorzugt, die 0 bis 8,0, insbesondere 2,0 bis 6,0 und besonders bevorzugt 3,0 bis 6,0 Gew.-% P₂O₅ enthält. P₂O₅ kann insbesondere als Keimbildner für die Bildung von Lithiumsilikat fungieren. Das Vorhandensein eines Keimbildners ist für die Bildung von Lithiumsilikat als Hauptkristallphase jedoch nicht zwingend erforderlich.

Auch ist es bevorzugt, dass die Glaskeramik neben Li₂O weiteres Alkalimetalloxid Me^{I}₂O in einer Menge von 0 bis 10,0, insbesondere 0,5 bis 8,0 und besonders bevorzugt 1,0 bis 5,0 Gew.-% enthält. Der Begriff "weiteres Alkalimetalloxid Me^{I}₂O" bezeichnet Alkalimetalloxid mit Ausnahme von Li₂O, wobei dieses Me^{I}₂O insbesondere ausgewählt ist aus Na₂O, K₂O, Rb₂O und/oder Cs₂O. Besonders bevorzugt enthält die Glaskeramik mindestens eines und insbesondere alle der folgenden weiteren Alkalimetalloxide Me^{I}₂O in den angegebenen Mengen:

| Komponente | Gew.-% |
|---|---|
| Na₂O | 0 bis 3,0, insbesondere 0 bis 2,0 |
| K₂O | 0 bis 5,0 |
| Rb₂O | 0 bis 3,0, insbesondere 0 bis 2,0 |
| Cs₂O | 0 bis 10,0, insbesondere 0 bis 8,0. |

In einer besonders bevorzugten Ausführungsform enthält die erfindungsgemäß verwendete Glaskeramik 0,1 bis 5,0 und insbesondere 0,5 bis 4,5 Gew.-% K₂O.

Des Weiteren ist es bevorzugt, dass die Glaskeramik 0 bis 10,0 und insbesondere 2,0 bis 7,0 Gew.-% weiteres Oxid zweiwertiger Elemente Me^{II}O enthält. Der Begriff "weiteres Oxid zweiwertiger Elemente Me^{II}O" bezeichnet zweiwertige Oxide mit Ausnahme von CaO und MgO, wobei dieses Me^{II}O insbesondere ausgewählt ist aus SrO und/oder ZnO. Besonders bevorzugt enthält die Glaskeramik mindestens eines und insbesondere alle der folgenden weiteren Oxide zweiwertiger Elemente Me^{II}O in den angegebenen Mengen:

| Komponente | Gew.-% |
|---|---|
| SrO | 0 bis 5,0 |
| ZnO | 0 bis 5,0. |

Es ist weiter eine Glaskeramik bevorzugt, die 0 bis 10,0, bevorzugt 0 bis 8,0 und insbesondere 2,0 bis 5,0 Gew.-% Oxid dreiwertiger Elemente Me^{III}₂O₃ enthält, wobei dieses Me^{III}₂O₃ insbesondere ausgewählt ist aus Al₂O₃, B₂O₃, Y₂O₃, La₂O₃, Ga₂O₃ und/oder In₂O₃. Besonders bevorzugt enthält die Glaskeramik mindestens eines und insbesondere alle der folgenden Oxide dreiwertiger Elemente Me^{III}₂O₃ in den angegebenen Mengen:

| Komponente | Gew.-% |
|---|---|
| Al₂O₃ | 0 bis 8,0 |
| Y₂O₃ | 0 bis 5,0 |
| B₂O₃ | 0 bis 4,0 |
| Ga₂O₃ | 0 bis 5,0 |
| In₂O₃ | 0 bis 5,0 |
| La₂O₃ | 0 bis 5,0. |

In einer besonders bevorzugten Ausführungsform enthält die erfindungsgemäß verwendete Glaskeramik 0,1 bis 8,0, insbesondere 1,0 bis 7,0 Gew.-% und besonders bevorzugt 2,0 bis 5,0 Gew.-% Al₂O₃.

Ferner ist eine Glaskeramik bevorzugt, die weiteres Oxid vierwertiger Elemente Me^{IV}O₂ in einer Menge von 0 bis 15,0 Gew.-% und bevorzugt 0 bis 10,0 Gew.-% enthält. Der Begriff "weiteres Oxid vierwertiger Elemente Me^{IV}O₂" bezeichnet vierwertige Oxide mit Ausnahme von SiO₂, wobei dieses Me^{IV}O₂ insbesondere ausgewählt ist aus ZrO₂, GeO₂, CeO₂, TiO₂ und/oder SnO₂. Besonders bevorzugt enthält die Glaskeramik mindestens eines und insbesondere alle der folgenden weiteren Oxide vierwertiger Elemente Me^{IV}O₂ in den angegebenen Mengen:

| Komponente | Gew.-% |
|---|---|
| ZrO₂ | 0 bis 7,0 |
| TiO₂ | 0 bis 5,0 |
| SnO₂ | 0 bis 5,0 |
| GeO₂ | 0 bis 14,0 |
| CeO₂ | 0 bis 2,0. |

Außerdem ist eine Glaskeramik bevorzugt, die weiteres Oxid fünfwertiger Elemente Me^{V}₂O₅ in einer Menge von 0 bis 4,0 und insbesondere 0 bis 3,0 Gew.-% enthält. Der Begriff "weiteres Oxid fünfwertiger Elemente Me^{V}₂O₅" bezeichnet fünfwertige Oxide mit Ausnahme von P₂O₅, wobei dieses Me^{V}₂O₅ insbesondere ausgewählt ist aus V₂O₅, Ta₂O₅ und/oder Nb₂O₅. Besonders bevorzugt enthält die Glaskeramik mindestens eines und insbesondere alle der folgenden weiteren Oxide fünfwertiger Elemente Me^{V}₂O₅ in den angegebenen Mengen:

| Komponente | Gew.-% |
|---|---|
| V₂O₅ | 0 bis 1,0 |
| Ta₂O₅ | 0 bis 3,0 |
| Nb₂O₅ | 0 bis 3,0. |

Auch ist eine Glaskeramik bevorzugt, die 0 bis 5,0 Gew.-% Oxid sechswertiger Elemente Me^{VI}O₃ enthält, wobei dieses Me^{VI}O₃ insbesondere ausgewählt ist aus WO₃ und/oder MoO₃. Besonders bevorzugt enthält die Glaskeramik mindestens eines und insbesondere alle der folgenden Oxide Me^{VI}O₃ in den angegebenen Mengen:

| Komponente | Gew.-% |
|---|---|
| WO₃ | 0 bis 5,0 |
| MoO₃ | 0 bis 5,0. |

Weiterhin ist eine Glaskeramik bevorzugt, die 0 bis 3,0 und insbesondere 0 bis 1,0 Gew.-% Fluor enthält.

Besonders bevorzugt ist eine Glaskeramik, die mindestens eine und bevorzugt alle der folgenden Komponenten in den angegebenen Mengen enthält:

| Komponente | Gew.-% |
|---|---|
| SiO₂ | 53,0 bis 75,0 |
| Li₂O | 10,0 bis 23,0 |
| CaO | 1,0 bis 13,0 |
| MgO | 1,0 bis 12,0 |
| P₂O₅ | 0 bis 8,0 |
| Me^{I}₂O | 0 bis 10,0 |
| Me^{II}O | 0 bis 10,0 |
| Me^{III}₂O₃ | 0 bis 10,0 |
| Me^{IV}O₂ | 0 bis 15,0 |
| Me^{V}₂O₅ | 0 bis 4, 0 |
| Me^{VI}O₃ | 0 bis 5,0 |
| Fluor | 0 bis 3,0. |

wobei Me^{I}₂O, Me^{II}O, Me^{III}₂O₃, Me^{IV}O₂, Me^{V}₂O₅ und Me^{VI}O₃ die oben angegebene Bedeutung haben.

Manche der vorstehend genannten Komponenten können als Färbemittel und/oder Fluoreszenzmittel dienen. Die erfindungsgemäß verwendete Glaskeramik kann darüber hinaus noch weitere Färbemittel und/oder Fluoreszenzmittel enthalten, die insbesondere aus anorganischen Pigmenten und/oder Oxiden von d- und f-Elementen, wie z.B. den Oxiden von Sc, Mn, Fe, Co, Pr, Nd, Tb, Er, Dy, Gd, Eu und Yb, ausgewählt sein können. Als weitere Färbemittel können auch Metallkolloide, z.B. von Ag, Au und Pd, verwendet werden, die zusätzlich auch als Keimbildner fungieren können. Diese Metallkolloide können z.B. durch Reduktion von entsprechenden Oxiden, Chloriden oder Nitraten während der Schmelz- und Kristallisationsprozesse gebildet werden.

Die Eigenschaften der Glaskeramik werden maßgeblich durch die Kristallphasen beeinflusst. Die erfindungsgemäß verwendete Glaskeramik enthält Lithiumsilikat als Hauptkristallphase. Der Begriff "Lithiumsilikat" bezeichnet mindestens eine Kristallphase ausgewählt aus Lithiumdisilikat und Lithiummetasilikat. Mithin enthält die erfindungsgemäß verwendete Glaskeramik Lithiumdisilikat, Lithiummetasilikat oder eine Mischung von Lithiumdisilikat und Lithiummetasilikat als Hauptkristallphase. In einer bevorzugten Ausführungsform enthält die erfindungsgemäß verwendete Glaskeramik Lithiumdisilikat als Hauptkristallphase.

Mit dem Begriff "Hauptkristallphase" wird die Kristallphase bezeichnet, die von allen in der Glaskeramik vorhandenen Kristallphasen den höchsten Massenanteil hat. Die Bestimmung der Massen der Kristallphasen erfolgt dabei insbesondere mit der Rietveld-Methode. Ein geeignetes Verfahren zur quantitativen Analyse der Kristallphasen mittels der Rietveld-Methode ist z.B. in der Dissertation von M. Dittmer "Gläser und Glaskeramiken im System MgO-Al₂O₃-SiO₂ mit ZrO₂ als Keimbildner", Universität Jena 2011, beschrieben.

Es ist weiter bevorzugt, dass die erfindungsgemäß verwendete Glaskeramik 10,0 bis 75,0 und insbesondere 20,0 bis 75,0 Gew.-% Lithiumsilikat als Kristallphase enthält. Insbesondere ist es bevorzugt, dass die Glaskeramik 20,0 bis 75,0 und insbesondere 25,0 bis 60,0 Gew.-% Lithiumdisilikat und/oder 10,0 bis 60,0 und insbesondere 20,0 bis 50,0 Gew.-% Lithiummetasilikat als Kristallphase enthält.

Die erfindungsgemäß verwendete Glaskeramik enthält zusätzlich zu Lithiumsilikat als Hauptkristallphase noch Diopsid als weitere Kristallphase. In einer bevorzugten Ausführungsform enthält die Glaskeramik 0,1 bis 50,0, insbesondere 0,1 bis 25,0, besonders bevorzugt 0,1 bis 7,0 und ganz besonders bevorzugt 0,1 bis 5,0 Gew.-% Diopsid.

Die erfindungsgemäß verwendete Glaskeramik kann ferner weitere Kristallphasen, wie beispielsweise Li₃PO₄, SiO₂-Modifikationen, Enstatit und/oder Cs_{0, 809}AlSi₅O₁₂ enthalten.

Die Art und die Menge der gebildeten Kristallphasen können insbesondere durch die Zusammensetzung des Ausgangsglases sowie das Verfahren zur Herstellung der Glaskeramik gesteuert werden. Die Beispiele veranschaulichen dies anhand der Variation der Zusammensetzung und der Herstellungsverfahren.

Es wurde überraschenderweise gefunden, dass eine Glaskeramik bereitgestellt werden kann, die neben einer Diopsid-Kristallphase auch eine Lithiumsilikat-Kristallphase aufweist. Dabei war es insbesondere nicht vorhersehbar, dass eine solche Glaskeramik in dem oben beschriebenen bevorzugten Zusammensetzungsbereich gebildet werden kann. Es wurde gefunden, dass die Keimbildung und das Wachstum beider Kristallphasen offenbar nebeneinander in dem Ausgangsglas ablaufen. Dabei waren im Volumen des Ausgangsglases Lithiumsilikat-Kristalle, hingegen an der Oberfläche des Ausgangsglases Diopsid-Kristalle feststellbar. Danach scheint im Volumen des Ausgangsglases Keimbildung und Wachstum von Lithiumsilikat-Kristallen und demgegenüber an der Oberfläche des Ausgangsglases Keimbildung und Wachstum von Diopsid-Kristallen aufzutreten. Eine Kristallisation im Volumen eines Glases wird in der Fachwelt auch als Volumenkristallisation und eine Kristallisation an der Oberfläche wird auch als Oberflächenkristallisation bezeichnet.

Die Keimbildung und Kristallisation an der Oberfläche findet bei der Herstellung der erfindungsgemäß verwendeten Glaskeramik jedoch nicht ohne weiteres statt. Vielmehr wurde gefunden, dass es notwendig ist, die Oberfläche des Ausgangsglases zu aktivieren, indem es gemahlen wird. Durch diese spezielle Aktivierung gelingt eine reproduzierbare Oberflächenkristallisation von Diopsid. Dabei kann die Art und Weise des Mahlens, zum Beispiel die Verwendung von unterschiedlichen Mühlen, Einfluss auf die Menge an schließlich kristallisiertem Diopsid haben.

Die Diopsid-Menge in der erfindungsgemäß verwendeten Glaskeramik wird damit nicht durch zum Beispiel den Gehalt an MgO und CaO im Ausgangsglas oder dessen Wärmebehandlung, sondern auch durch die Art und Weise der Aktivierung infolge Mahlens des Ausgangsglases bestimmt.

Des Weiteren wurde gefunden, dass die Menge an ausgeschiedenem Diopsid einen Einfluss auf die Transluzenz der erfindungsgemäß verwendeten Glaskeramik hat. Durch einen Diopsid-Gehalt von insbesondere mehr als 5,0 Gew.-% können stark getrübte Glaskeramiken mit einem Kontrastwert (CR-Wert gemäß British Standard BS 5612) von mehr als 90 erzeugt werden. Diese Glaskeramiken eignen sich insbesondere zur Herstellung einer dentalen Abutmentstruktur oder einer dentalen Suprastruktur, auf die eine geeignete Verblendung aufgebracht wird.

Durch einen verhältnismäßig geringen Diopsid-Gehalt von insbesondere weniger als 5,0 und bevorzugt weniger als 2,0 Gew.-% können transluzente Glaskeramiken mit einem CR-Wert von weniger als 75 erzeugt werden. Diese Glaskeramiken eignen sich insbesondere zur Herstellung von optisch anspruchsvollen Dentalrestaurationen, wie Kronen, Verblendungen und Inlays.

Die erfindungsgemäß verwendete Glaskeramik zeichnet sich weiter dadurch aus, dass sie sogar nach abschließender Ausbildung der der Glaskeramik eine hohe Festigkeit verleihenden Lithiumdisilikat-Kristallphase gut maschinell bearbeitbar ist, um sie z.B. in die Form einer Dentalrestauration zu bringen. Dies ist ein besonderer Vorteil gegenüber konventionellen Lithiumdisilikat-Glaskeramiken, bei denen häufig eine maschinell einfacher bearbeitbare Vorstufe verwendet wird und diese Vorstufe nach der maschinellen Bearbeitung noch einer Wärmebehandlung zur Bildung der gewünschten Lithiumdisilikat-Glaskeramik unterzogen werden muss.

Die erfindungsgemäß verwendete Glaskeramik zeichnet sich auch durch eine sehr gute chemische Beständigkeit aus. Zur Bestimmung der chemischen Beständigkeit wurde die Glaskeramik gemäß ISO-Norm 6872 (2008) geprüft, indem der Masseverlust bei Lagerung in wässriger Essigsäure bestimmt wurde. Die erfindungsgemäß verwendete Glaskeramik zeigte dabei einen Masseverlust von vorzugsweise weniger als 100 µg/cm².

Die erfindungsgemäß verwendete Glaskeramik weist zudem eine biaxiale Bruchfestigkeit σ_{B} von vorzugsweise mindestens 200 MPa und besonders bevorzugt 200 bis 400 MPa auf. Die biaxiale Bruchfestigkeit wurde gemäß ISO 6872 (2008) (Kolben-auf-drei-Kugeln-Prüfung) bestimmt.

Mithin bietet die erfindungsgemäß verwendete Glaskeramik eine wünschenswerte Kombination von vorteilhaften optischen und mechanischen Eigenschaften, wie sie insbesondere für ein Dentalmaterial angestrebt wird.

Die Erfindung betrifft ebenfalls die Verwendung von Vorstufen mit entsprechender Zusammensetzung, aus denen die erfindungsgemäß verwendete Glaskeramik durch Wärmebehandlung hergestellt werden kann. Diese Vorstufen sind ein entsprechend zusammengesetztes Ausgangsglas und ein entsprechend zusammengesetztes Ausgangsglas mit Keimen. Die Bezeichnung "entsprechender Zusammensetzung" bedeutet, dass diese Vorstufen die gleichen Komponenten in den gleichen Mengen wie die Glaskeramik enthalten, wobei die Komponenten mit Ausnahme von Fluor als Oxide berechnet werden, so wie es bei Gläsern und Glaskeramiken üblich ist.

Die Erfindung betrifft daher ebenfalls die Verwendung eines Ausgangsglases, das die Komponenten der erfindungsgemäß verwendeten Lithiumsilikat-Diopsid-Glaskeramik enthält. Es sind alle solche Ausführungsformen für die Komponenten des Ausgangsglases bevorzugt, die auch für die Komponenten der erfindungsgemäß verwendeten Lithiumsilikat-Diopsid-Glaskeramik als bevorzugt angegeben sind.

Besonders bevorzugt liegt das Ausgangsglas in gemahlener Form oder in Form eines aus gemahlenem Ausgangsglas gepressten Pulverpresslings vor. In diesen beiden Formen hat das Ausgangsglas durch das Mahlen eine Aktivierung erfahren, die für die spätere Kristallisation von Diopsid erforderlich ist.

Die Erfindung betrifft weiter auch ein Ausgangsglas, das Keime für die Kristallisation von Lithiumsilikat und/oder Diopsid enthält.

Gegenstand der Erfindung ist auch eine Lithiumsilikat-Diopsid-Glaskeramik, die Lithiumsilikat als Hauptkristallphase und Diopsid als weitere Kristallphase enthält und 2,0 bis 8,0 Gew.-% P₂O₅ enthält. Weiterer Gegenstand der Erfindung ist ein Ausgangsglas, das die Komponenten der Glaskeramik enthält und insbesondere Keime für die Kristallisation von Lithiummetasilikat, Lithiumdisilikat und/oder Diopsid enthält. Dabei sind für die Glaskeramik und das Ausgangsglas alle Ausführungsformen bevorzugt, die für die erfindungsgemäß verwendete Lithiumsilikat-Diopsid-Glaskeramik und das erfindungsgemäß verwendete Ausgangsglas als bevorzugt angegeben sind.

Ferner betrifft die Erfindung ein Verfahren zur Herstellung der erfindungsgemäßen Lithiumsilikat-Diopsid-Glaskeramik, bei dem
(a) Ausgangsglas gemahlen wird,
(b) gegebenenfalls das gemahlene Ausgangsglas zu einem Pulverpressling verpresst wird und
(c) das gemahlene Ausgangsglas oder der Pulverpressling mindestens einer Wärmebehandlung bei einer Temperatur im Bereich von 500° bis 1000°C für eine Dauer von 5 bis 120 min unterzogen wird.

In der Stufe (a) wird das erfindungsgemäße Ausgangsglas gemahlen, um es für die Kristallisation von Diopsid zu aktivieren.

Das Mahlen erfolgt insbesondere in Mühlen und bevorzugt in Kugelmühlen, Strahlmühlen, wie Gegenstrahlmühlen, oder Schwingmühlen. Die nach dem Mahlen erhaltenen Glasteilchen haben üblicherweise eine mittlere Teilchengröße im Bereich von 100 nm bis 100 µm, bezogen auf die Anzahl der Teilchen.

Durch die Verwendung unterschiedlicher Mahlverfahren, z.B. durch Einsatz unterschiedlicher Mühlen, kann ein unterschiedlicher Grad an Aktivierung des Ausgangsglases erzielt und damit auch die Menge an schließlich kristallisiertem Diopsid gesteuert werden.

Das dem Mahlvorgang unterworfene Ausgangsglas liegt vorzugsweise in Form eines Granulates vor. Dabei wird mit dem Begriff "Granulat" ein teilchenförmiges Ausgangsglas bezeichnet. Zur Erzeugung von teilchenförmigem Ausgangsglas kann eine Schmelze des Ausgangsglases in Wasser eingegossen und damit abgeschreckt werden. Dieser Vorgang wird auch als Fritten und das erhaltene Glasgranulat als Glasfritte bezeichnet. Ein Granulat kann aber auch auf andere Weise, wie zum Beispiel durch Abschrecken in einem Walzenstuhl und anschließende Zerkleinerung erzeugt werden.

Die Herstellung des Ausgangsglases erfolgt insbesondere in der Weise, dass eine Mischung von geeigneten Ausgangsmaterialien, wie z.B. Carbonaten, Oxiden, und Phosphaten, bei Temperaturen von insbesondere 1300 bis 1700°C, bevorzugt bei etwa 1500 °C, für eine Dauer von 0,5 bis 5 h erschmolzen wird.

In der optionalen Stufe (b) wird das gemahlene Ausgangsglas zu einem Pulverpressling verpresst. Es ist bevorzugt, dass in dem erfindungsgemäßen Verfahren diese Stufe durchgeführt wird.

Im Gegensatz zu einem Glasmonolithen, wie er z.B. durch Gießen einer Glasschmelze in eine Form erhalten wird, zeichnet sich der erfindungsgemäße Pulverpressling durch eine hohe innere Oberfläche aus, an der Kristallisation von Diopsid erfolgen kann.

Der Pulverpressling kann eine beliebige Geometrie aufweisen. Üblicherweise hat der Pulverpressling bereits im Wesentlichen die Form, die für einen Rohling aus der später erzeugten erfindungsgemäßen Glaskeramik vorgesehen ist.

In der Stufe (c) wird das gemahlene Glas oder der Pulverpressling mindestens einer Wärmebehandlung unterworfen. Diese mindestens eine Wärmebehandlung erfolgt bei einer Temperatur im Bereich von 500° bis 1000°C, vorzugsweise 700° bis 1000°C, bevorzugt 750° bis 950°C und besonders bevorzugt 800° bis 950°C für eine Dauer von 5 bis 120 min, vorzugsweise 5 bis 90 min.

Die Wärmebehandlung wird durchgeführt, bis die gewünschte Menge an Lithiumsilikat und Diopsid kristallisiert ist und damit die erfindungsgemäße Lithiumsilikat-Diopsid-Glaskeramik gebildet worden ist. Die Wärmebehandlung kann auch stufenweise erfolgen, wobei durch eine erste Wärmebehandlung zunächst eine Vorstufe, wie keimgebildetes Ausgangsglas, und dann durch eine zweite Wärmebehandlung bei einer höheren Temperatur die erfindungsgemäße Glaskeramik gebildet wird. Die Bildung von Keimen für die Kristallisation von Lithiumsilikat findet dabei üblicherweise bei einer Temperatur im Bereich von 450 bis 600°C statt.

Es ist weiter bevorzugt, die Wärmebehandlung so zu wählen, dass es auch zu einem wenigstens teilweisen Sintern, d.h. einem Vorsintern, des gemahlenen Ausgangsglases oder des Pulverpresslings kommt. Besonders bevorzugt ist es, wenn die Wärmebehandlung auch zu einem möglichst vollständigen Sintern, d.h. einem Dichtsintern des gemahlenen Ausgangsglases oder des Pulverpresslings führt.

Aus gemahlenem Ausgangsglas erzeugte dichtgesinterte Glaskeramiken finden dabei vor allem als Beschichtungen auf Substraten, wie dentalen Suprastrukturen, Anwendung. Aus Pulverpresslingen erzeugte dichtgesinterte Glaskeramiken werden vor allem als Rohlinge eingesetzt, aus denen durch geeignete Formgebungsverfahren wie Pressen und insbesondere maschinelle Bearbeitung Dentalrestaurationen, wie Brücken, Kronen, Inlays oder Onlays, hergestellt werden können.

Nach Abschluss von Stufe (c) liegt die erfindungsgemäße Lithiumsilikat-Diopsid-Glaskeramik vor.

Aus der erfindungsgemäß verwendeten Glaskeramik und den erfindungsgemäß verwendeten Gläsern können dentale Restaurationen, wie Brücken, Inlays, Onlays, Kronen, Veneers, Schalen oder Abutments, hergestellt werden. Die Erfindung betrifft daher deren Verwendung als Dentalmaterial und insbesondere deren Verwendung zur Herstellung dentaler Restaurationen. Dabei ist es bevorzugt, dass der Glaskeramik oder dem Glas durch Verpressen oder maschinelle Bearbeitung die Form der gewünschten dentalen Restauration gegeben wird.

Das Verpressen erfolgt üblicherweise unter erhöhtem Druck und erhöhter Temperatur. Es ist bevorzugt, dass das Verpressen bei einer Temperatur von 700 bis 1200°C erfolgt. Weiter ist es bevorzugt, das Verpressen bei einem Druck von 10 bis 30 bar durchzuführen. Beim Verpressen wird durch viskoses Fließen des eingesetzten Materials die gewünschte Formänderung erreicht. Dabei können die erfindungsgemäß verwendeten Gläser und Glaskeramiken insbesondere in Form von Rohlingen in beliebiger Form und Größe eingesetzt werden. Für das Verpressen wird bevorzugt die erfindungsgemäß verwendete Glaskeramik verwendet.

Die maschinelle Bearbeitung erfolgt üblicherweise durch materialabtragende Verfahren und insbesondere durch Fräsen und/oder Schleifen. Es ist besonders bevorzugt, dass die maschinelle Bearbeitung im Rahmen eines CAD/CAM-Verfahrens durchgeführt wird. Dabei können die erfindungsgemäß verwendeten Gläser und Glaskeramiken insbesondere in Form von Rohlingen eingesetzt werden. Diese sind von ihrer Form regelmäßig an den Typ der für die maschinelle Bearbeitung eingesetzten Maschine angepasst. Für die maschinelle Bearbeitung wird insbesondere die erfindungsgemäß verwendete Glaskeramik verwendet.

Aufgrund der vorstehend geschilderten Eigenschaften der erfindungsgemäß verwendeten Glaskeramiken und der erfindungsgemäß verwendeten Gläser eignen sich diese insbesondere zum Einsatz in der Zahnheilkunde. Gegenstand der Erfindung ist daher auch die Verwendung der Glaskeramiken oder der Gläser als Dentalmaterial und insbesondere zur Herstellung dentaler Restaurationen, wie Kronen, Brücken und Abutments.

Die Erfindung betrifft mithin auch ein Verfahren zur Herstellung einer dentalen Restauration, insbesondere Brücke, Inlay, Onlay, Veneer, Abutment, Teilkrone, Krone oder Schale, bei dem der erfindungsgemäß verwendeten Glaskeramik oder dem erfindungsgemäß verwendeten Glas durch Verpressen oder durch maschinelle Bearbeitung, insbesondere im Rahmen eines CAD/CAM-Verfahrens, die Form der gewünschten dentalen Restauration gegeben wird.

Die Erfindung betrifft weiter ein Verfahren zur Beschichtung eines Substrats, bei dem gemahlenes Ausgangsglas auf das Substrat aufgebracht und kristallisiert sowie gesintert wird. Die Kristallisation und Sinterung erfolgt unter den Bedingungen, wie sie oben für die Wärmebehandlung gemäß Schritt (c) des erfindungsgemäßen Verfahrens angegeben sind. Als Substrate kommen insbesondere Oxidkeramiken oder Glaskeramiken infrage. Geeignete Oxidkeramiken sind Al₂O₃- oder ZrO₂-Keramiken sowie Mischungen davon, z.B. teil- oder vollstabilisierte ZrO₂-Keramik mit Gehalten an MgO, CaO, Y₂O₃ und/oder CeO₂. Geeignete Glaskeramiken sind Lithiumsilicat-Glaskeramiken oder Glaskeramiken vom SiO₂-Al₂O₃-K₂O-Typ.

Die Erfindung wird im Folgenden anhand von sie nichtbeschränkenden Beispielen näher erläutert.

### Beispiele

### Beispiele 1 bis 22 - Zusammensetzung und Kristallphasen

Es wurden insgesamt 22 Gläser und Glaskeramiken mit der in Tabelle I angegebenen Zusammensetzung hergestellt.

Dabei bedeuten in Tabelle I:
- T_{g}: Glasübergangstemperatur, bestimmt mittels DSC
- T_{S} und t_{S}: Angewendete Temperatur und Zeit für Erschmelzung des Ausgangsglases
- T_{Sinter} und t_{Sinter}: Angewendete Temperatur und Zeit für die Wärmebehandlung und damit Kristallisation von Presslingen
- T_{Press} und t_{Press}: Angewendete Temperatur und Zeit für das Verpressen von kristallisierten Presslingen
- L*a*b-Wert: Schlüssel zur Charakterisierung der Farbe
- CR-Wert: Kontrastwerts der Glaskeramik gemäß British Standard BS 5612
- Li₂Si₂O₅: Lithiumdisilikat
- Li₂SiO3: Lithiummetasilikat
- CaMgSi₂O₆: Diopsid
- SiO₂: Quarz, insbesondere Tiefquarz, oder Cristobalit
- Cs_{0.809}AlSi₅O₁₂: Cäsiumalumosilicat

In den Beispielen 1 bis 22 wurden Gläser aus üblichen Rohstoffen in einem Platintiegel bei der Temperatur T_{S} für eine Dauer t_{S} erschmolzen. Durch Eingießen der erschmolzenen Ausgangsgläser in Wasser wurden Glasfritten, d.h. Glasgranulate, hergestellt. Für die Weiterverarbeitung der Glasfritten wurden die nachstehend angegebenen drei Verfahrensvarianten A), B) und C) benutzt:

### A) Schwingmühlen

Die gemäß den Beispielen 1 bis 9, 11 bis 19, 21 und 22 hergestellten Glasfritten wurden mit einer Schwingmühle KM100 der Firma Retsch GmbH, Haan, Deutschland, und einer Zirkonoxidschwingmühle RM31 der Firma Retsch GmbH, Haan, Deutschland auf eine mittlere Korngröße von <90 µm, bezogen auf die Anzahl der Teilchen gemahlen. Das gemahlene Glaspulver wurde anschließend uniaxial zu einem kleinen Zylinder verpresst und in einem Ofen vom Typ Programat (Ivoclar Vivadent AG) bei der Temperatur T_{Sinter} für die Dauer t_{Sinter} kristallisiert und gesintert. An den hergestellten Prüfkörpern wurden Röntgenbeugungsanalysen zur Bestimmung der vorhandenen Kristallphasen sowie Farbmessungen durchgeführt.

### B) Strahlmühle

Die Glasfritte mit der Zusammensetzung gemäß Beispiel 10 wurde in einer Gegenstrahlmühle AFG 100, der Firma Hosokawa Alpine, auf eine mittlere Korngröße von 20µm, bezogen auf die Anzahl der Teilchen gemahlen. Das gemahlene Glaspulver wurde daraufhin uniaxial verpresst und in einem Ofen vom Typ Programat (Ivoclar Vivadent AG) bei der Temperatur T_{Sinter} für die Dauer t_{Sinter} kristallisierte und gesintert. An den so hergestellten Prüfkörpern wurden Farbmessungen sowie Röntgenbeugungsanalysen durchgeführt. Der CR-Wert der erzeugten Lithiumsilikat-Diopsid-Glaskeramik betrug 69,95.

### C) Kugelmühle

Die Glasfritte mit der Zusammensetzung gemäß Beispiel 20 wurde in einer Kugelmühle für eine Dauer von ca. 20h auf eine mittlere Korngröße von 10µm, bezogen auf die Anzahl der Teilchen, gemahlen. Die Kugelmühle hatte als Mahlraum einen zylinderförmigen Porzellanbehälter mit einem Fassungsvermögen von 5 l. Als Mahlkörper wurde die folgende Mischung aus Porzellanmahlkugeln verwendet: 0,9 kg mit Durchmesser 10 mm, 1,8 kg mit Durchmesser 20 mm und 0,9 kg mit Durchmesser 30 mm. Das gemahlene Glaspulver wurde dann uniaxial verpresst und in einem Ofen vom Typ Programat (Ivoclar Vivadent AG) bei der Temperatur T_{Sinter} für die Dauer t_{Sinter} kristallisiert und gesintert. An den so hergestellten Prüfkörpern wurden Farbmessungen sowie Röntgenbeugungsanalysen zur Bestimmung der Kristallphasen durchgeführt. Der Gehalt an Diopsid-Kristallen war bei dieser Glaskeramik höher als bei den nach Varianten A) und B) hergestellten Glaskeramiken.

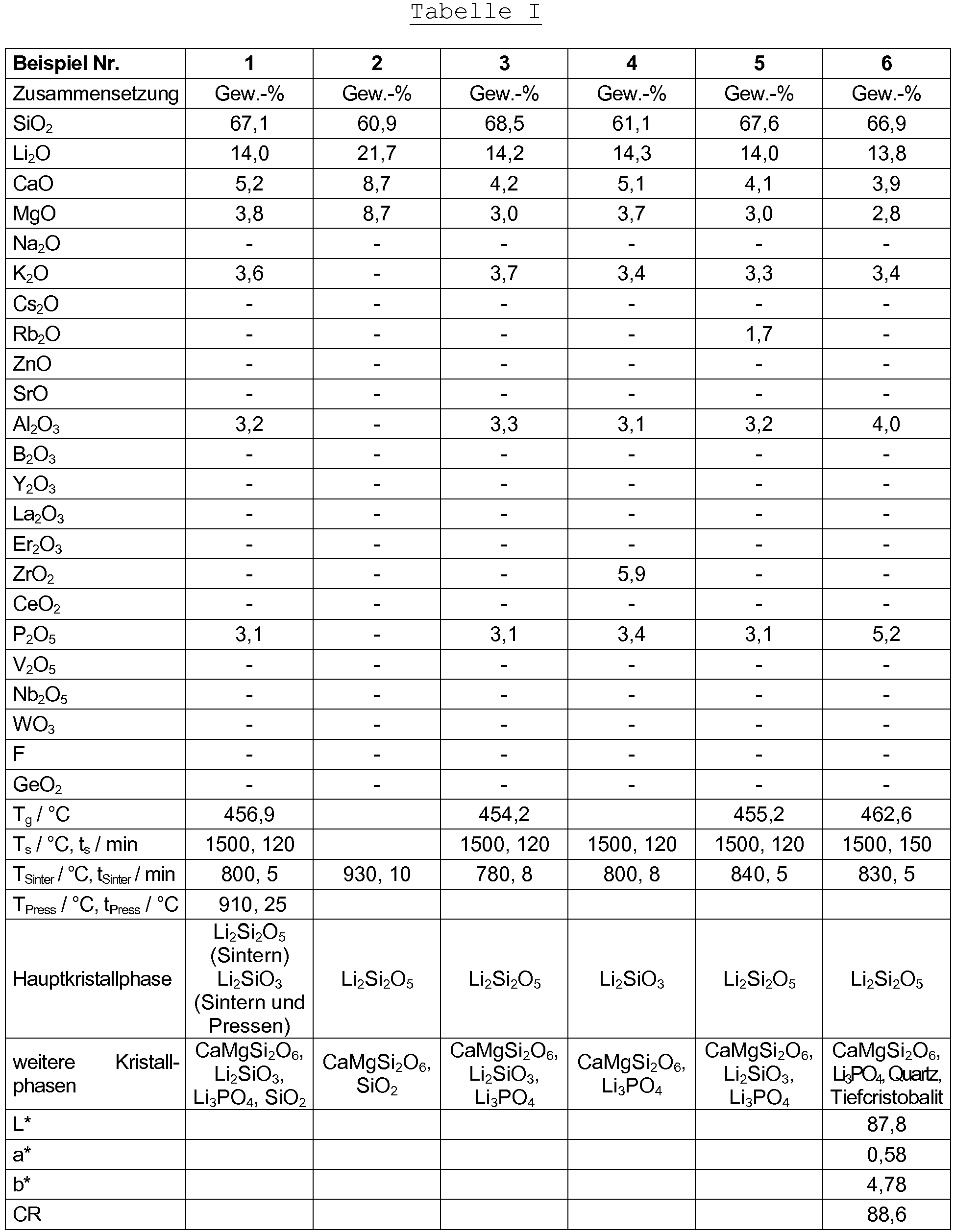

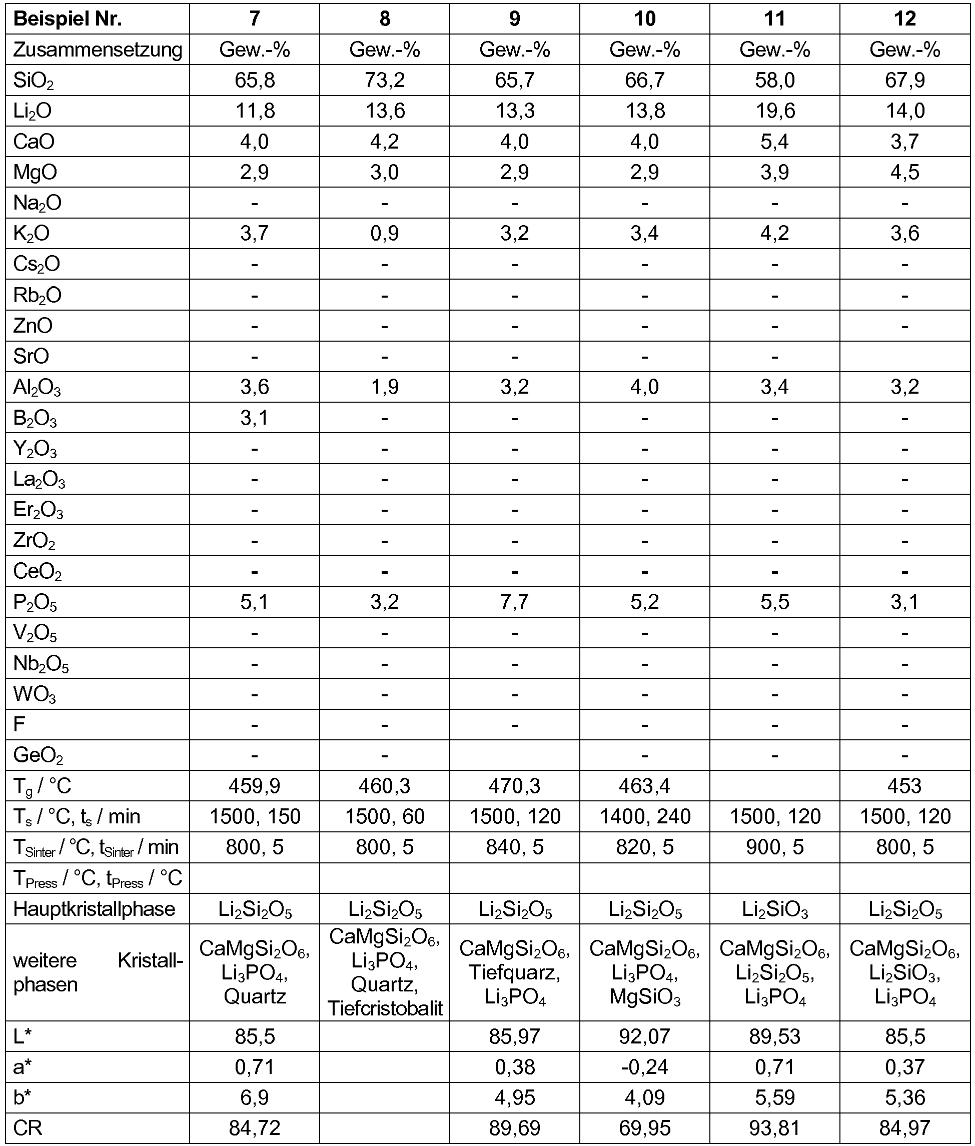

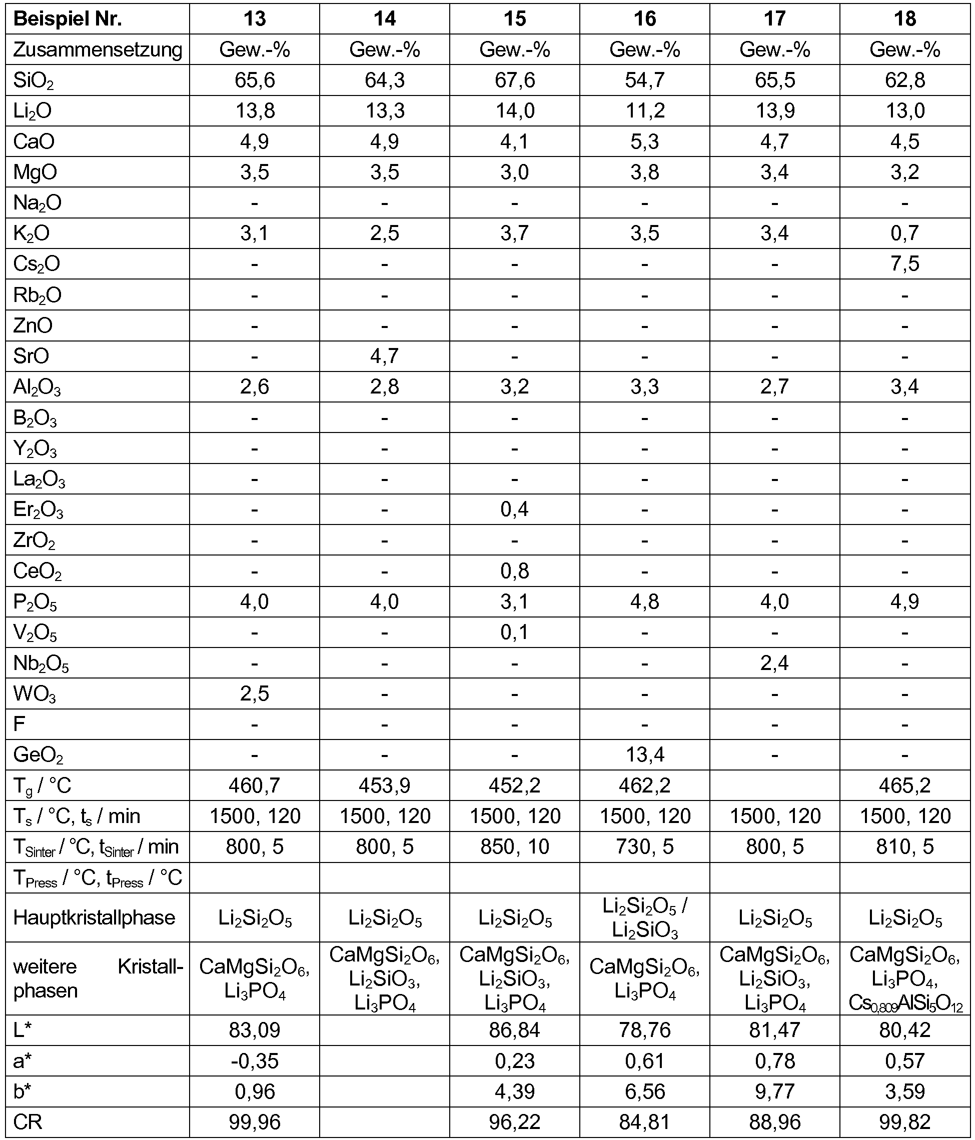

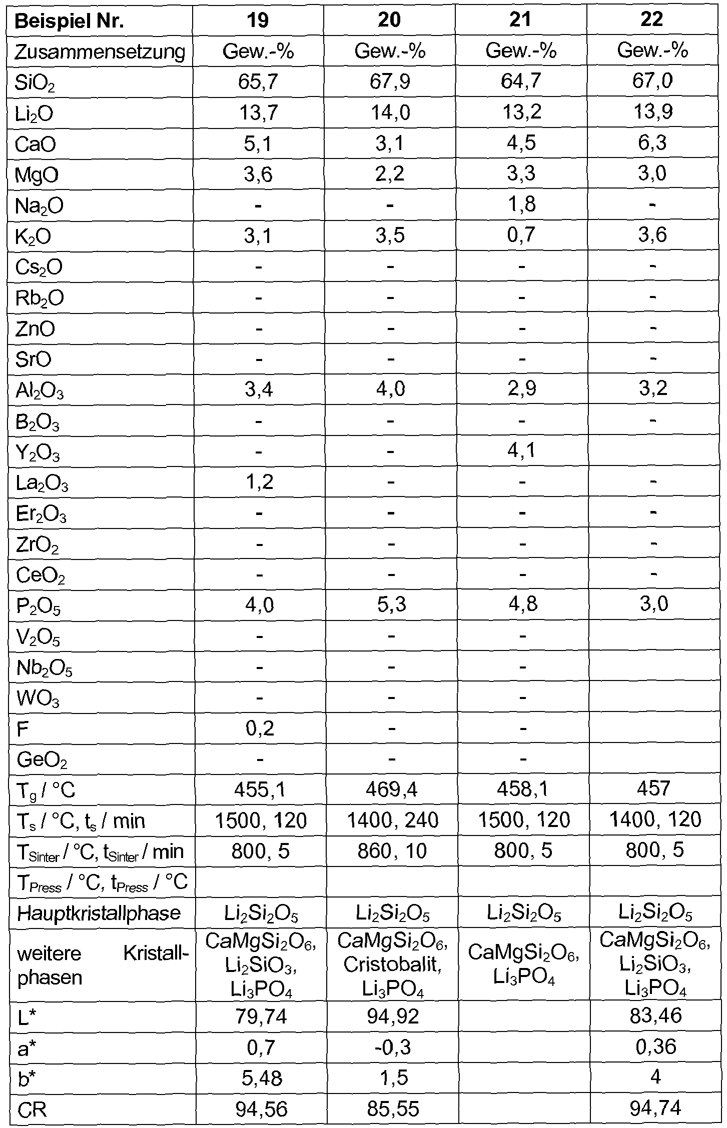

### Beispiel 23 - Einfluss der Zerkleinerung

Eine Glasfritte mit der Zusammensetzung gemäß Beispiel 10 wurde mit einer Kugelmühle analog wie für Beispiel 20 angegeben auf eine mittlere Korngröße von 20µm, bezogen auf die Anzahl der Teilchen, gemahlen. Das gemahlene Glaspulver wurde daraufhin uniaxial verpresst und in einem Ofen vom Typ Programat (Ivoclar Vivadent AG) bei einer Temperatur von 870°C für eine Dauer von 5 min kristallisiert und gesintert. An dem so hergestellten Prüfkörper wurden anschließend eine Farbmessung (Minolta-Apparatur) und eine Röntgenbeugungsanalyse zur Kristallphasenbestimmung durchgeführt. Li₂Si₂O₅ bildete die Hauptkristallphase der Glaskeramik. Diopsid und Li₃PO₄ waren die Nebenkristallphasen. Der Diopsid-gehalt war größer als in Beispiel 10. Der erhöhte Diopsidanteil führt zu einem höheren Trübungsgrad, was an einem CR-Wert von 90,00 anstelle von 69,95 abzulesen war.

### Beispiel 24 - Heißpressen

Ein Glas mit der Zusammensetzung gemäß Beispiel 1 wurde in einem Platintiegel bei einer Temperatur von 1500°C erschmolzen und anschließend in Wasser gegossen. Die so hergestellte Glasfritte wurde mit einer Schwingmühle KM100 der Firma Retsch GmbH, Haan, Deutschland, auf eine mittlere Korngröße von <90µm, bezogen auf die Anzahl der Teilchen, gemahlen. Aus dem erhaltenen Glaspulver wurde durch uniaxiales Pressen ein Pulverpressling hergestellt. Der Pulverpressling wurde bei einer Temperatur von 800°C und einer Haltezeit von 5 min in einem Ofen vom Typ Programat kristallisiert und dichtgesintert. Der kristallisierte und dichtgesinterte Rohling wurde anschließend durch Heißpressen bei einer Haltezeit von 25 min bei einer Temperatur von 910°C verpresst. An den verpressten Probekörpern wurde eine Röntgenstrukturanalyse durchgeführt, und der thermische Ausdehnungskoeffizient sowie die die Biaxialfestigkeit des verpressten Materials wurde nach ISO 6872 bestimmt. Die Biaxialfestigkeit betrug 230 MPa.

### Mechanische Bearbeitbarkeit

Zum Test der mechanischen Bearbeitbarkeit wurden Glaspulver gemäß den Beispielen 3, 7, 10, 12, 16, 18 und 23 uniaxial zu Blöcken verpresst und in einem Ofen vom Typ Programat dichtgesintert. Auf die so hergestellten Glaskeramikblöcke wurden daraufhin entsprechende Halter aufgeklebt und sie wurden mit einer CAD/CAM-Schleifeinheit (Sirona InLab) bearbeitet. Zum Test der Bearbeitbarkeit wurden dabei Biaxialprüfkörper aus den Blöcken geschliffen.

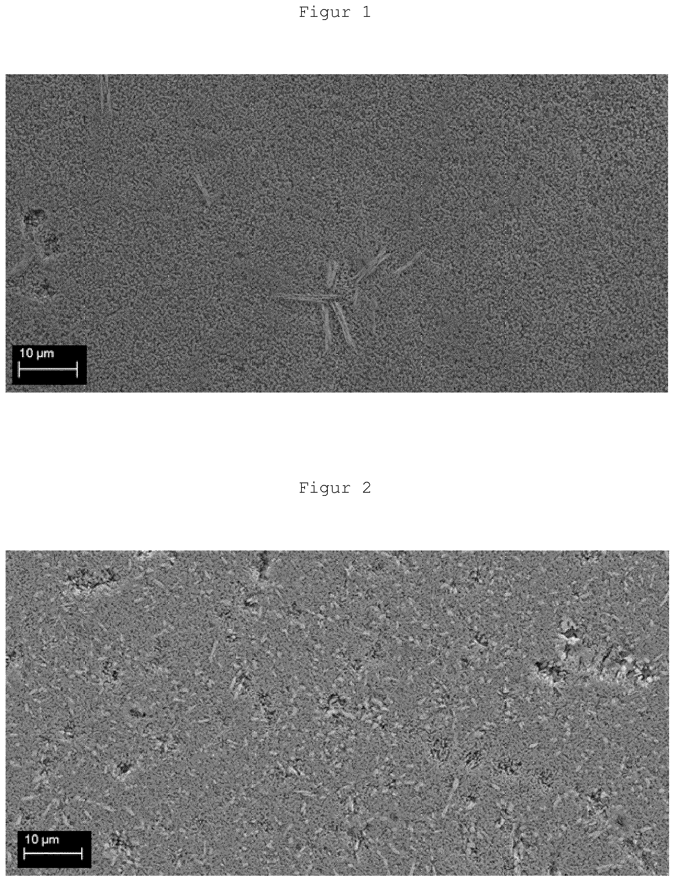

### Figuren 1 und 2 - Gefügebilder

Figur 1 zeigt das Gefüge der Glaskeramik gemäß Beispiel 10. Kennzeichnend ist das sehr feine Lithiumdisilikatgefüge mit wenigen dazwischenliegenden Diopsid-Kristallen. Figur 2 zeigt das Gefüge der gemäß Beispiel 23 erhaltenen Glaskeramik, und es ist deutlich die gegenüber Beispiel 10 gesteigerte Bildung von Diopsid erkennbar.

## Patentansprüche

1. Verwendung einer Lithiumsilikat-Diopsid-Glaskeramik, die Lithiumsilikat als Hauptkristallphase und Diopsid als weitere Kristallphase enthält, als Dentalmaterial und insbesondere zur Herstellung dentaler Restaurationen.

2. Verwendung nach Anspruch 1, bei der die Glaskeramik 53,0 bis 75,0, insbesondere 54,0 bis 74,0 und bevorzugt 58,0 bis 70,0 Gew.-% SiO₂ enthält.

3. Verwendung nach Anspruch 1 oder 2, bei der die Glaskeramik 10,0 bis 23,0, insbesondere 11,0 bis 20,0 und bevorzugt 11,0 bis 16,0 Gew.-% Li₂O enthält.

4. Verwendung nach einem der Ansprüche 1 bis 3, bei der die Glaskeramik 1,0 bis 13,0, insbesondere 1,0 bis 9,0 und bevorzugt 1,0 bis 6,0 Gew.-% CaO und/oder 1,0 bis 12,0, insbesondere 2,0 bis 9,0 und bevorzugt 2,0 bis 5,0 Gew.-% MgO enthält.

5. Verwendung nach Anspruch 4, wobei das molare Verhältnis von CaO zu MgO insbesondere 0,5 bis 2,0, bevorzugt 0,8 bis 1,2 und besonders bevorzugt etwa 1,0 beträgt.

6. Verwendung nach einem der Ansprüche 1 bis 5, bei der die Glaskeramik 0 bis 8,0, insbesondere 2,0 bis 6,0 und bevorzugt 3,0 bis 6,0 Gew.-% P₂O₅ enthält.

7. Verwendung nach einem der Ansprüche 1 bis 6, bei der die Glaskeramik 0 bis 10,0, bevorzugt 0,5 bis 8,0 und besonders bevorzugt 1,0 bis 5,0 Gew.-% weiteres Alkalimetalloxid Me^{I}₂O enthält, wobei Me^{I}₂O insbesondere ausgewählt ist aus Na₂O, K₂O, Rb₂O und/oder Cs₂O.

8. Verwendung nach einem der Ansprüche 1 bis 7, bei der die Glaskeramik 0 bis 10,0 und bevorzugt 2,0 bis 7,0 Gew.-% weiteres Oxid zweiwertiger Elemente Me^{II}O enthält, wobei Me^{II}O insbesondere ausgewählt ist aus SrO und/oder ZnO.

9. Verwendung nach einem der Ansprüche 1 bis 8, bei der die Glaskeramik 0 bis 10,0, bevorzugt 0 bis 8,0 und besonders bevorzugt 2,0 bis 5,0 Gew.-% Oxid dreiwertiger Elemente Me^{III}₂O₃ enthält, wobei Me^{III}₂O₃ insbesondere ausgewählt ist aus Al₂O₃, B₂O₃, Y₂O₃, La₂O₃ Ga₂O₃ und/oder In₂O₃ und insbesondere Al₂O₃ ist.

10. Verwendung nach einem der Ansprüche 1 bis 9, bei der die Glaskeramik 0 bis 15,0 und bevorzugt 0 bis 10,0 Gew.-% weiteres Oxid vierwertiger Elemente Me^{IV}O₂ enthält, wobei Me^{IV}O₂ insbesondere ausgewählt ist aus ZrO₂, GeO₂, CeO₂, TiO₂ und/oder SnO₂.

11. Verwendung nach einem der Ansprüche 1 bis 10, bei der die Glaskeramik 0 bis 4,0 und bevorzugt 0 bis 3,0 Gew.-% weiteres Oxid fünfwertiger Elemente Me^{V}₂O₅ enthält, wobei Me^{V}₂O₅ insbesondere ausgewählt ist aus V₂O₅, Ta₂O₅ und/oder Nb₂O₅.

12. Verwendung nach einem der Ansprüche 1 bis 11, bei der die Glaskeramik 0 bis 5,0 Gew.-% Oxid sechswertiger Elemente Me^{VI}O₃ enthält, wobei Me^{VI}O₃ insbesondere WO₃ und/oder MoO₃ ist.

13. Verwendung nach einem der Ansprüche 1 bis 12, bei der die Glaskeramik mindestens eine und bevorzugt alle folgenden Komponenten enthält:
| Komponente | Gew.-% |
|---|---|
| SiO₂ | 53,0 bis 75,0 |
| Li₂O | 10,0 bis 23,0 |
| CaO | 1,0 bis 13,0 |
| MgO | 1,0 bis 12,0 |
| P₂O₅ | 0 bis 8,0 |
| Me^{I}₂O | 0 bis 10,0 |
| Me^{II}O | 0 bis 10,0 |
| Me^{III}₂O₃ | 0 bis 10,0 |
| Me^{IV}O₂ | 0 bis 15,0 |
| Me^{V}₂O₅ | 0 bis 4,0 |
| Me^{VI}O₃ | 0 bis 5,0 |
| Fluor | 0 bis 3,0. |

14. Verwendung nach einem der Ansprüche 1 bis 13, bei der die Glaskeramik 10,0 bis 75,0 Gew.-%, bevorzugt 20,0 bis 75,0 Gew.-% an Lithiumsilikat-Kristallen aufweist.

15. Verwendung nach einem der Ansprüche 1 bis 14, bei der die Glaskeramik Lithiumsilikat in Form von Lithiumdisilikat und/oder Lithiummetasilikat enthält.

16. Verwendung nach Anspruch 15, bei der die Glaskeramik 20,0 bis 75,0 Gew.-%, insbesondere 25,0 bis 60,0 Gew.-% an Lithiumdisilikat-Kristallen aufweist.

17. Verwendung nach Anspruch 15 oder 16, bei der die Glaskeramik 10,0 bis 60,0 Gew.-%, insbesondere 20,0 bis 50,0 Gew.-% an Lithiummetasilikat-Kristallen aufweist.

18. Verwendung nach einem der Ansprüche 1 bis 17, bei der die Glaskeramik 0,1 bis 50,0, insbesondere 0,1 bis 25,0, bevorzugt 0,1 bis 7,0 und ganz besonders bevorzugt 0,1 bis 5,0 Gew.-% an Diopsid-Kristallen aufweist.

19. Verwendung zur Herstellung dentaler Restaurationen nach einem der Ansprüche 1 bis 18, wobei der Glaskeramik durch Verpressen oder maschinelle Bearbeitung die Form der gewünschten dentalen Restauration, insbesondere Brücke, Inlay, Onlay, Veneer, Abutment, Teilkrone, Krone oder Schale, gegeben wird.

20. Verwendung eines Ausgangsglases, das die Komponenten der Glaskeramik nach einem der Ansprüche 1 bis 13 enthält und insbesondere Keime für die Kristallisation von Lithiummetasilikat, Lithiumdisilikat und/oder Diopsid enthält, als Dentalmaterial und insbesondere zur Herstellung dentaler Restaurationen.

21. Lithiumsilikat-Diopsid-Glaskeramik, die Lithiumsilikat als Hauptkristallphase und Diopsid als weitere Kristallphase enthält und 2,0 bis 8,0 Gew.-% P₂O₅ enthält.

22. Glaskeramik nach Anspruch 21, die in Form von einem Rohling oder einer dentalen Restauration vorliegt.

23. Ausgangsglas, das die Komponenten der Glaskeramik nach Anspruch 21 enthält und insbesondere Keime für die Kristallisation von Lithiummetasilikat, Lithiumdisilikat und/oder Diopsid enthält.

24. Ausgangsglas nach Anspruch 23, das in Form von einem gemahlenen Pulver oder einem Pressling aus gemahlenem Pulver vorliegt.

25. Verfahren zur Herstellung der Glaskeramik gemäß Anspruch 21 oder 22, bei dem
(a) das Ausgangsglas gemäß Anspruch 23 gemahlen wird,
(b) gegebenenfalls das gemahlene Ausgangsglas zu einem Pulverpressling verpresst wird und
(c) das gemahlene Ausgangsglas oder der Pulverpressling mindestens einer Wärmebehandlung bei einer Temperatur im Bereich von 500° bis 1000°C für eine Dauer von 5 bis 120 min unterzogen wird.

## Claims

1. Use of a lithium silicate-diopside glass ceramic, which comprises lithium silicate as main crystal phase and diopside as further crystal phase, as dental material and in particular for the preparation of dental restorations.

2. Use according to claim 1, wherein the glass ceramic comprises 53.0 to 75.0, in particular 54.0 to 74.0 and preferably 58.0 to 70.0 wt.-% SiO₂.

3. Use according to claim 1 or 2, wherein the glass ceramic comprises 10.0 to 23.0, in particular 11.0 to 20.0 and preferably 11.0 to 16.0 wt.-% Li₂O.

4. Use according to any one of claims 1 to 3, wherein the glass ceramic comprises 1.0 to 13.0, in particular 1.0 to 9.0 and preferably 1.0 to 6.0 wt.-% CaO and/or 1.0 to 12.0, in particular 2.0 to 9.0 and preferably 2.0 to 5.0 wt.-% MgO.

5. Use according to claim 4, wherein the molar ratio of CaO to MgO is in particular 0.5 to 2.0, preferably 0.8 to 1.2 and particularly preferred about 1.0.

6. Use according to any one of claims 1 to 5, wherein the glass ceramic comprises 0 to 8.0, in particular 2.0 to 6.0 and preferably 3.0 to 6.0 wt.-% P₂O₅.

7. Use according to any one of claims 1 to 6, wherein the glass ceramic comprises 0 to 10.0, preferably 0.5 to 8.0 and more preferably 1.0 to 5.0 wt.-% further alkali metal oxide Me^{I}₂O, wherein Me^{I}₂O is selected in particular from Na₂O, K₂O, Rb₂O and/or Cs₂O.

8. Use according to any one of claims 1 to 7, wherein the glass ceramic comprises 0 to 10.0 and preferably 2.0 to 7.0 wt.-% further oxide of divalent elements Me^{II}O, wherein Me^{II}O is selected in particular from SrO and/or ZnO.

9. Use according to any one of claims 1 to 8, wherein the glass ceramic comprises 0 to 10.0, preferably 0 to 8.0 and particularly preferred 2.0 to 5.0 wt.-% further oxide of trivalent elements Me^{III}₂O₃, wherein Me^{III}₂O₃ is selected in particular from Al₂O₃, B₂O₃, Y₂O₃, La₂O₃ Ga₂O₃ and/or In₂O₃ and in particular is Al₂O₃.

10. Use according to any one of claims 1 to 9, wherein the glass ceramic comprises 0 to 15.0 and preferably 0 to 10.0 wt.-% further oxide of tetravalent elements Me^{IV}O₂, wherein Me^{IV}O₂ is selected in particular from ZrO₂, GeO₂, CeO₂, TiO₂ and/or SnO₂.

11. Use according to any one of claims 1 to 10, wherein the glass ceramic comprises 0 to 4.0 and preferably 0 to 3.0 wt.-% further oxide of pentavalent elements Me^{V}₂O₅, wherein Me^{V}₂O₅ is selected in particular from V₂O₅, Ta₂O₅ and/or Nb₂O₅.

12. Use according to any one of claims 1 to 11, wherein the glass ceramic comprises 0 to 5.0 wt.-% oxide of hexavalent elements Me^{VI}O₃, wherein Me^{VI}O₃ is in particular WO₃ and/or MoO₃.

13. Use according to any one of claims 1 to 12, wherein the glass ceramic comprises at least one and preferably all of the following components:
| Component | wt.-% |
|---|---|
| SiO₂ | 53.0 to 75.0 |
| Li₂O | 10.0 to 23.0 |
| CaO | 1.0 to 13.0 |
| MgO | 1.0 to 12.0 |
| P₂O₅ | 0 to 8.0 |
| Me^{I}₂O | 0 to 10.0 |
| Me^{II}O | 0 to 10.0 |
| Me^{III}₂O₃ | 0 to 10.0 |
| Me^{IV}O₂ | 0 to 15.0 |
| Me^{V}₂O₅ | 0 to 4.0 |
| Me^{VI}O₃ | 0 to 5.0 |
| fluorine | 0 to 3.0. |

14. Use according to any one of claims 1 to 13, wherein the glass ceramic has 10.0 to 75.0 wt.-%, preferably 20.0 to 75.0 wt.-% lithium silicate crystals.

15. Use according to any one of claims 1 to 14, wherein the glass ceramic comprises lithium silicate in the form of lithium disilicate and/or lithium metasilicate.

16. Use according to claim 15, wherein the glass ceramic has 20.0 to 75.0 wt.-%, in particular 25.0 to 60.0 wt.-% lithium disilicate crystals.

17. Use according to claim 15 or 16, wherein the glass ceramic has 10.0 to 60.0 wt.-%, in particular 20.0 to 50.0 wt.-% lithium metasilicate crystals.

18. Use according to any one of claims 1 to 17, wherein the glass ceramic has 0.1 to 50.0, in particular 0.1 to 25.0, preferably 0.1 to 7.0 and particularly preferred 0.1 to 5.0 wt.-% diopside crystals.

19. Use for the preparation of dental restorations according to any one of claims 1 to 18, wherein the glass ceramic is given the shape of the desired dental restoration, in particular bridge, inlay, onlay, veneer, abutment, partial crown, crown or facet, by pressing or machining.

20. Use of a starting glass, which comprises the components of the glass ceramic according to any one of claims 1 to 13 and in particular nuclei for the crystallization of lithium metasilicate, lithium disilicate, and/or diopside, as dental material and in particular for the preparation of dental restorations.

21. Lithium silicate-diopside glass ceramic, which comprises lithium silicate as main crystal phase and diopside as further crystal phase and 2.0 to 8.0 wt.-% P₂O₅.

22. Glass ceramic according to claim 21, which is present in the form of a blank or a dental restoration.

23. Starting glass, which comprises the components of the glass ceramic according to claim 21 and in particular nuclei for the crystallization of lithium metasilicate, lithium disilicate and/or diopside.

24. Starting glass according to claim 23, which is present in the form of a ground powder or a compact made of ground powder.

25. Process for the preparation of the glass ceramic according to claim 21 or 22, comprising
(a) grinding the starting glass according to claim 23,
(b) optionally pressing the ground starting glass to form a powder compact and
(c) subjecting the ground starting glass or the powder compact to at least one heat treatment at a temperature in the range of 500 to 1000°C for a period of 5 to 120 min.

## Revendications

1. Utilisation, en tant que matériau dentaire et en particulier pour la fabrication de pièces de restauration dentaire, d'une vitrocéramique de silicate de lithium et de diopside qui contient du silicate de lithium en tant que principale phase cristalline et du diopside en tant qu'autre phase cristalline.

2. Utilisation conforme à la revendication 1, dans laquelle la vitrocéramique contient de 53,0 à 75,0, en particulier de 54,0 à 74,0 et de préférence de 58,0 à 70,0 % en poids de SiO₂.

3. Utilisation conforme à la revendication 1 ou 2, dans laquelle la vitrocéramique contient de 10,0 à 23,0, en particulier de 11,0 à 20,0 et de préférence de 11,0 à 16,0 % en poids de Li₂O.

4. Utilisation conforme à l'une des revendications 1 à 3, dans laquelle la vitrocéramique contient de 1,0 à 13,0, en particulier de 1,0 à 9,0 et de préférence de 1,0 à 6,0 % en poids de CaO et/ou de 1,0 à 12,0, en particulier de 2,0 à 9,0 et de préférence de 2,0 à 5,0 % en poids de MgO.

5. Utilisation conforme à la revendication 4, dans laquelle le rapport molaire de CaO à MgO vaut en particulier de 0,5 à 2,0, de préférence de 0,8 à 1,2 et mieux encore environ 1,0.

6. Utilisation conforme à l'une des revendications 1 à 5, dans laquelle la vitrocéramique contient de 0 à 8,0, en particulier de 2,0 à 6,0 et de préférence de 3,0 à 6,0 % en poids de P₂O₅.

7. Utilisation conforme à l'une des revendications 1 à 6, dans laquelle la vitrocéramique contient de 0 à 10,0, de préférence de 0,5 à 8,0 et mieux encore de 1,0 à 5,0 % en poids d'autres oxydes de métaux alcalins Me^{I}₂O, étant entendu que Me^{I}₂O est choisi en particulier parmi Na₂O, K₂O, Rb₂O et/ou Cs₂O.

8. Utilisation conforme à l'une des revendications 1 à 7, dans laquelle la vitrocéramique contient de 0 à 10,0 et de préférence de 2,0 à 7,0 % en poids d'autres oxydes d'éléments divalents Me^{II}O, étant entendu que Me^{II}O est choisi en particulier parmi SrO et/ou ZnO.

9. Utilisation conforme à l'une des revendications 1 à 8, dans laquelle la vitrocéramique contient de 0 à 10,0, de préférence de 0 à 8,0 et mieux encore de 2,0 à 5,0 % en poids d'oxydes d'éléments trivalents Me^{III}₂O₃, étant entendu que Me^{III}₂O₃ est choisi en particulier parmi Al₂O₃, B₂O₃, Y₂O₃, La₂O₃, Ga₂O₃ et/ou In₂O₃, et est plus particulièrement Al₂O₃.

10. Utilisation conforme à l'une des revendications 1 à 9, dans laquelle la vitrocéramique contient de 0 à 15,0 et de préférence de 0 à 10,0 % en poids d'autres oxydes d'éléments tétravalents Me^{IV}O₂, étant entendu que Me^{IV}O₂ est choisi en particulier parmi ZrO₂, GeO₂, CeO₂, TiO₂ et/ou SnO₂.

11. Utilisation conforme à l'une des revendications 1 à 10, dans laquelle la vitrocéramique contient de 0 à 4,0 et de préférence de 0 à 3,0 % en poids d'autres oxydes d'éléments pentavalents Me^{V}₂O₅, étant entendu que Me^{V}₂O₅ est choisi en particulier parmi V₂O₅, Ta₂O₅ et/ou Nb₂O₅.

12. Utilisation conforme à l'une des revendications 1 à 11, dans laquelle la vitrocéramique contient de 0 à 5,0 % en poids d'oxydes d'éléments hexavalents Me^{VI}O₃, étant entendu que Me^{VI}O₃ est en particulier WO₃ et/ou MoO₃.

13. Utilisation conforme à l'une des revendications 1 à 12, dans laquelle la vitrocéramique contient au moins l'un des composants suivants, et de préférence, les contient tous :
| Composant | % en poids |
|---|---|
| SiO₂ | 53,0 - 75,0 |
| Li₂O | 10,0 - 23,0 |
| CaO | 1,0 - 13,0 |
| MgO | 1,0 - 12,0 |
| P₂O₅ | 0 - 8,0 |
| Me^{I}₂O | 0 - 10,0 |
| Me^{II}O | 0 - 10,0 |
| Me^{III}₂O₃ | 0 - 10,0 |
| Me^{IV}O₂ | 0 - 15,0 |
| Me^{V}₂O₅ | 0 - 4,0 |
| Me^{VI}O₃ | 0 - 5,0 |
| Fluor | 0 - 3,0 |

14. Utilisation conforme à l'une des revendications 1 à 13, dans laquelle la vitrocéramique comporte de 10,0 à 75,0 % en poids et de préférence de 20,0 à 75,0 % en poids de cristaux de silicate de lithium.

15. Utilisation conforme à l'une des revendications 1 à 14, dans laquelle la vitrocéramique contient du silicate de lithium sous forme de disilicate de lithium et/ou de métasilicate de lithium.

16. Utilisation conforme à la revendication 15, dans laquelle la vitrocéramique comporte de 20,0 à 75,0 % en poids et en particulier de 25,0 à 60,0 % en poids de cristaux de disilicate de lithium.

17. Utilisation conforme à la revendication 15 ou 16, dans laquelle la vitrocéramique comporte de 10,0 à 60,0 % en poids et en particulier de 20,0 à 50,0 % en poids de cristaux de métasilicate de lithium.

18. Utilisation conforme à l'une des revendications 1 à 17, dans laquelle la vitrocéramique comporte de 0,1 à 50,0, en particulier de 0,1 à 25,0, de préférence de 0,1 à 7,0 et surtout de 0,1 à 5,0 % en poids de cristaux de diopside.

19. Utilisation pour la fabrication de pièces de restauration dentaire, conforme à l'une des revendications 1 à 18, dans laquelle on donne à la vitrocéramique, par compression ou travail à la machine, la forme des pièces de restauration dentaire voulues, en particulier bridges, incrustations inlay ou onlay, facettes « veneer », piliers, couronnes partielles, couronnes ou plaquettes.

20. Utilisation, en tant que matériau dentaire et en particulier pour la fabrication de pièces de restauration dentaire, d'un verre de départ qui contient les constituants d'une vitrocéramique définie dans l'une des revendications 1 à 13, et qui contient en particulier des germes pour la cristallisation du métasilicate de lithium, du disilicate de lithium et/ou du diopside.

21. Vitrocéramique de silicate de lithium et de diopside, qui contient du silicate de lithium en tant que principale phase cristalline et du diopside en tant qu'autre phase cristalline, et qui contient de 2,0 à 8,0 % en poids de P₂O₅.

22. Vitrocéramique conforme à la revendication 21, qui se présente sous la forme d'une ébauche ou d'une pièce de restauration dentaire.

23. Verre de départ qui contient les constituants d'une vitrocéramique conforme à la revendication 21, et qui contient en particulier des germes pour la cristallisation du métasilicate de lithium, du disilicate de lithium et/ou du diopside.

24. Verre de départ conforme à la revendication 23, qui se présente sous la forme d'une poudre moulue ou d'un comprimé obtenu à partir d'une poudre moulue.

25. Procédé de fabrication d'une vitrocéramique conforme à la revendication 21 ou 22, dans lequel
a) on moud un verre de départ conforme à la revendication 23,
b) en option, on comprime le verre de départ moulu pour en faire un comprimé de poudre,
c) et l'on soumet le verre de départ moulu ou le comprimé de poudre à au moins un traitement thermique, à une température située dans l'intervalle allant de 500 à 1000 °C et durant un laps de temps de 5 à 120 minutes.
